# EUROPEAN PATENT APPLICATION

(11) **EP 3 764 367 A1**
(43) Date of publication of application: **13.01.2021**
(21) Application number: 19185624.4
(22) Date of filing: 11.07.2019
(51) Int. Cl.: G16H 30/40, G16H 40/63

(54) **SYSTEM AND METHOD FOR MEDICAL GROSS EXAMINATION**

(71) Applicant: Milestone S.r.l., 24010 Sorisole (BG) (IT)
(72) Inventor: Visinoni, Francesco, 24030 Mozzo (BG) (IT); Bellini, Marco, 24128 Bergamo (IT); Salvi, Mattia, 24040 Stezzano (BG) (IT)
(74) Representative: Kiwit, Benedikt

(57) **Abstract**

The present invention relates to a system (100) for medical gross examination. The system (100) comprises an examination area (200) for receiving biospecimens (TS), a camera device (300) for visual inspection of the examination area (200) and for providing images captured during visual inspection, and a display device (400) for displaying the captured images. The system (100) further comprises an eye-tracking device (500) for detecting eye information based on the point of gaze (505) or eye movement of a user's (OP) eye and a controller (600) for controlling the system (100) based on the detected eye information.

Furthermore, the invention relates to a method for the control of a system (100) for medical gross examination, comprising the steps of placing a biospecimen (TS) in an examination area (200), visually inspecting the examination area (200), capturing images thereof and displaying the captured images. The method further comprises the steps of detecting eye information based on the point of gaze (505) or eye movement of a user (OP) and controlling the system (100) based on the detected eye information.

## Description

The present invention relates to a system for medical gross examination, comprising an examination area for receiving biological specimens and a camera device for visual inspection of the examination area. The present invention further relates to a method for the control of a system for medical gross examination.

The term "gross examination" or "grossing" refers to a process, in which (bio-) specimens for pathology analysis are inspected, measured and documented to obtain diagnostic information, as well as processed for further microscopic examination.

Gross examination of surgical specimens is typically performed by a pathologist or by a pathologist's assistant working within a pathology practice. The individuals trained in this field are often able to obtain critical information for diagnosis of illnesses by performing gross examination. For example, it is possible to determine the stage of a surgically removed tumor and the status (class) of the margin around the tumor by gross examination. Based on this information a surgeon may be able to decide if a tumor has been completely removed or if further surgical resection of affected tissue has to be conducted. Sometimes such decisions have to be made by the surgeon within a relatively short time. Consequently, the gross examination has to be completed within a reasonable time.

Typically, two end products will be produced during gross examination:
The first end product, which is called "gross description", is a document that serves as written record of the examiner's findings and is included in the final pathology report. The gross description comprises a visual description of the surgically excised specimen(s) and its sampling site. Selected high-resolution images can be used to supplement synoptic or preformatted reports that provide essential data or clinical relevance for the final pathological diagnose. The accuracy of the gross description depends, for example, on the amount, type and accuracy of information an operator can draw from the excised specimen in the analysis. To create the description, the operator will be required to use and to handle various pieces of equipment that support the analysis and reporting process, such as a camera or surgical instruments. Also, the operator may turn, lift and/or cut the specimen several times to investigate it. Often, the operator will use a dictating device, a keyboard or a pen to record findings and other information at different stages of its investigation.

The second end product is a set of blocks of tissue taken from the excised specimen. Each block of tissue is typically in the size of a postage stamp and sealed in plastic cassettes for further processing. The block can be processed into slides for microscopic examination. Chemical processing of the specimen, such as performing a fixation step for preserving the tissue from decay, may be involved in this process.

Thus, it becomes apparent that the gross examination involves a high amount of manual work and includes steps that require the operator to use and to operate equipment as well as to come into direct contact with the specimen.

However, gross examination typically creates a work environment that exposes operators to hazardous and contagious substances, which may be damaging their health. For example, such risks may arise from the specimen itself by carrying infectious diseases. Also, risks may arise from chemicals used during the gross examination. For example, fixation of biological tissue often requires usage of chemical substances, such as formaldehyde, that are harmful to human's health.

Previous attempts to address these risk factors include ventilation of a grossing station to prevent inhalation of infectious or formaldehyde fumes as well as providing the operator with personal protective equipment (PPE), such as disposable gloves, a lab coat and protective eye wear, to avoid the operator being in direct contact with chemicals and specimens.

A drawback of current grossing stations is that the equipment used during the gross examination has to be treated professionally after each procedure. The equipment may require cleaning and sterilizing to avoid contamination and spread of diseases. For example, the gloves worn during the examination of the specimen cannot be used for typing on a keyboard without contaminating it.

Continuously changing the PPE equipment in order to avoid extensive cleaning of the equipment has been found neither safe nor practical. In comparison, known solutions address this problem by providing only equipment that can be washed and sterilized either entirely or that comprises removable parts that are touched during the examination. However, this solution increases the initial costs for the system as well as for its upkeep while the availability rate of the system is reduced due to the interruptions induced by cleaning cycles. Moreover, over time the specialized equipment deteriorates quickly due to high temperatures and chemicals used for sterilizing. Also, the costs for staff are increased as the medically trained personal is involved in cleaning of the equipment and supervising the cleaning process.

Moreover, the documentation process that is an important part of the gross examination is slowed down by the professionals' duty to avoid contamination. In addition, the documentation process itself, as conducted in known solutions, allows still for improvement and more intuitive processing.

Thus, it is an object of the present invention to provide a system and a method for performing a gross examination that allow to obtain results of high accuracy, quality and reliability in a short period of time while maintaining the safety of the operator during gross examination.

The object is to be accomplished by means of the independent claims. The dependent claims advantageously study the central idea of the present invention further.

According to a first aspect of the present invention, a system for medical gross examination is provided. The system comprises an examination area for receiving biospecimens to be examined and a camera device. The camera device is suitable for visual inspection of the examination area and for providing images captured during visual inspection for transmission. The system further comprises a display device for displaying the captured images.

Therein, the term "biospecimen" may be understood as any (type and/or form of) biological specimen, such as (biological) tissue or organs of human or animal origin.

The camera device may provide the captured during visual inspection such that the captured images can be transmitted.

The system further comprises an eye-tracking device for detecting eye information based on the point of gaze and/or eye movement of a user's eye.

According to the present invention, the term "point of gaze" may be understood as a (temporary) location onto which the eye(s) of a user (=operator) are (momentarily) directed.

Moreover, the term "eye information" may be understood as any measurable parameter being (directly) related to a (momentary) state of the physiology of the human vision. The eye information may also include any further information derivable therefrom. For example, the eye information may comprise the size of the pupil opening or the direction in which the eyes are directed. This may allow, for example, to derive also information about the brightness of the surroundings or the point of gaze, respectively.

The system further comprises a controller for controlling the system based on the eye information detected by the eye-tracking device.

In other words, the present invention relates to a system that is suitable for conducting medical gross examination. Further, the system comprises elements, such as an examination area, a camera and a display, that allow for biospecimens placed on the examination area to be (visually) examined, (visually) depicted and (graphically) documented by an operator. In addition, the system comprises elements, such as the eye-tracking device and the controller, that allow for the system being controlled by (human) vision.

Thus, it can be achieved that the operator can reduce the amount of "manual" work. Instead, the operator's "hand work" centers on directly contacting the biospecimen as control and (manual) handling of equipment is accomplished by using eye information based control (which is done hands-free). Thus, the cleaning process and risk of contamination is significantly reduced in the system of the present invention. Also, the availability rates of the respective system are increased as cleaning cycles can be shortened. At the same time, the safety of operators and the environment can be maintained and improved. In addition, the operator can complete the gross examination in a shorter time period as frequent swaps between surgical instruments and documentation equipment can be avoided. Instead, the operator can focus on the analysis and description of the biospecimen. Thereby, the quality and reliability of the analysis results can be improved. Moreover, by providing the system according to the present invention costs for components, maintenance and upkeep can be reduced.

Also, the possibility to control a computer in a totally "hands free" manner through the use of a dedicated interface (such as eye control) is advantageous in environments where there is a high risk of contamination, where the use of a keyboard and a mouse could endanger the sterility of the system, or where hands are situated under a laboratory hood and occupied with surgical instruments. By providing an eye-tracking based application, a perceptual interface is created that replaces keyboard and mouse with an intuitive and efficient visual control, which enables facilitating the display, manipulation, analysis and sharing of medical images.

The system may further comprise at least one (additional) system component. Naturally, it is also conceivable that the system may comprise some or all of the following system components. Preferably, (each of) the system component(s) may be an element/part/device that is suitable for assisting the operator in performing gross examination.

For example, the system component may be a data system for storing gross examination data. Preferably, the gross examination data may be linked to a unique data code for storing (the gross examination data) in the data system. Alternatively or additionally, the system component may be an internet device for accessing external data or an external main data system. Alternatively or additionally, the system component may be a communication device, like a telephone device or a VoIP device, for (establishing) external communication. Alternatively or additionally, the system component may be a microphone device for recording an audio signal. Alternatively or additionally, the system component may be a ventilation device for ventilation of the examination area, for example by extracting or removing fumes from the examination area. Alternatively or additionally, the system component may be a robot arm device for handling biospecimens into and out of the examination area as well as for handling biospecimens at the examination area. The term "robot arm device" may be understood as any (type or form of) device suitable for assisting the user in the gross examination.

Thereby, the system is provided with various tools and equipment that assists the user/operator in their task of performing medical gross examination. Thereby, the results of the examination can be improved in quality and accuracy while reducing the time required for conducting the gross examination.

According to a preferred embodiment of the invention, the controller may be configured to activate defined control functions based on the eye information.

Generally, the expression of "activating a control function" may be understood as "selecting", "invoking", "calling", "triggering", "setting" and/or "operating" a control function.

Preferably, parameters of the defined control functions may be based on the detected eye information or set via the detected eye information.

Generally, the expression of "parameters of a control function" may be understood as assigning variables of the control function a defined value.

In other words, by providing the system with the above functionality the system can be controlled in a pre-defined manner and the control of the system can be tailored to a current (instantaneous/momentary) situation (during gross examination). Thus, the operator/user can interact with the system in a safe and deterministic manner that allows for a number of control options and being conducted in a hands-free manner.

Preferably, the defined control functions may comprise at least one function for the control of the system. Naturally, it is also conceivable that the defined control functions may comprise some or all of the following functions.

For example, the defined control functions may comprise a camera device function, such as a zoom-in/zoom-out function, an image capturing function, and/or a video recording function. Alternatively or additionally, the defined control functions may comprise a display device function, such as changing a resolution of the display device or content to be displayed, and/or editing the captured images. Alternatively or additionally, the defined control functions may comprise a data system function, such as accessing the data system for retrieving and storing data, e.g. (storing) data as displayed on the display device, and/or for searching and opening (patient) cases to be displayed on the display device. Alternatively or additionally, the defined control functions may comprise an internet access function for accessing external data (or an external main data system). Alternatively or additionally, the defined control functions may comprise a communication function for establishing a communication link. For example, the communication function may be used for calling a colleague or surgeon. Alternatively or additionally, the defined control functions may comprise a microphone device function, such as starting and stopping an audio recording. Alternatively or additionally, the defined control functions may comprise a robot arm function for manipulating/handling biospecimen and/or the camera device. Alternatively or additionally, the defined control functions may comprise a ventilation function for controlling the configuration of the ventilation, e.g. (setting) a power (level). Alternatively or additionally, the defined control functions may comprise a data analysis function for analyzing and evaluating data.

Thus, the operator/user can operate elements of the system entirely by using visual control. Thus, the gross examination process can be conducted in a timely and efficient manner without the need of having to directly contact the respective elements in order to operate them.

According to a preferred embodiment, the controller may be configured such that activating at least one of the control functions activates at least one other control function to be activated by the eye information. Preferably, the at least one activated control function may be displayed on the display device (for being operated by eye information). The activation and/or the display of the other activated control function may be for a predetermined time only.

Thus, by providing the system with the above-mentioned features it is possible to further specify functionalities or to interconnect/interact between functionalities such that synergetic effects can be created. For example, by activating the communication function also the microphone device function may be activated and operated (by eye information) to use the system's microphone for communicating with a colleague. Thus, ease of use is improved and time for operating the system can be saved. In particular, the operator is not required to touch or handle any equipment as it can be activated and operated by eye information. At the same time, the costs for the system can be reduced as some components can be used for multiple differing purposes.

Preferably, the controller may be configured to control or to allow control of at least one of the control functions or of the other control functions for a predetermined time only.

This allows the operator to reduce the time for controlling the system based on eye information so the focus of the operator can remain on conducting the examination.

According to a preferred embodiment, the controller may be configured such that the eye information is associated with a defined reference object, e.g. the display device.

Thus, the operator controls the system based on eye information that relate to a specific reference object. Consequently, the system can be controlled based on information relating to a physical reaction of the user to the reference object. Thereby, it can be achieved that the system control, data evaluation and documentation can be simplified. For example, by using the camera as a reference object, direct control of the camera can be effected without the need of pushing any buttons and the ease of control is improved. Thereby, the gross examination can be accelerated and improved.

Preferably, the controller may be configured such that the eye information is associated with dedicated areas of the reference object. Preferably, each of the dedicated areas may relate to at least one of the defined control functions of the system.

The dedicated areas may comprise at least one or all of the following (specific/pre-defined) areas: The dedicated areas may comprise defined spatial areas of the reference object, like a section of a raster. Alternatively or additionally, the dedicated areas may comprise defined areas on the display device, preferably divided in a raster having raster sections. Alternatively or additionally, the dedicated areas may comprise an image display area, preferably being divided into sub-areas, for displaying the captured image on the display device. Alternatively or additionally, the dedicated areas may comprise functional areas related to or displaying functions of the system to be operated, like control functions relating to the camera device, the display device, the data system, the internet device, the communication device, the microphone device, the robot arm device, and/or the ventilation device. Alternatively or additionally, the dedicated areas may comprise an input area for inputting and/or storing information in the system, preferably (in/via) the data system.

Moreover, the controller may be configured such that the control functions and/or the other activated control functions are displayed at the dedicated areas.

Thereby, the ease of control is improved and the gross examination can be accelerated. For example, activation of the various available tools can be accomplished by merely looking at their respective icons on the screen and selecting them accordingly. In addition, it is possible for the operator to customize its eye-tracking experience including a selection of the type of feedback of particular functions or the time allowed for an interaction with a particular function.

According to a preferred embodiment of the present invention, the controller may be configured to create a heat map based on the eye information. Preferably, the heat map may be based on detected eye positions as the eye information and being associated with relative or absolute durations recorded for each of the detected eye positions. Preferably, the eye positions may relate to the associated defined reference object, and more preferred to at least part of the dedicated areas.

Preferably, the controller may be configured to create the heat map with (different) colours for the respective dedicated areas, wherein different colours represent different durations or duration ranges recorded for the detected eye positions correlating with the dedicated areas.

Generally, the expression "eye positions" may relate to a position of the user's eye(s) itself or to (coordinates of) a position (in relation to a reference object) to which the eye(s) of the user are directed (focused on).

The system may be configured to record eye positions relating to (local) areas (of interest) of the biospecimen.

Thereby, it is possible to find a correlation between a position, onto which a user may focus, and a timespan that may, for example, indicate a magnitude of interest of the user in the respective position. This allows to improve the control of the system as well as utilizing the mental work performed by the operator during the examination. A skilled operator may recognize areas of interest quickly and the system supports and facilitates this analysis without interrupting the intellectual mental process of the operator by recording and determining areas that have "caught" the operator's eye(s). Known systems require to perform such analysis tasks twice, once during the (visual) examination and a second time during documentation. The present invention is capable of harvesting the mental work of user from the beginning.

Preferably, the controller may be configured to create the heat map based on the recorded duration of each of the detected eye positions with respect to the dedicated areas of the displayed captured image (of the biospecimen). Also, the detected eye positions may preferably relate to the dedicated areas of the biospecimen.

Thereby, it can be achieved to combine the analysis and examination process of the biospecimen directly with the documentation process. Thus, a significant time reduction for the gross examination can be achieved as relevant parts of the captured images can be highlighted and (automatically) prepared for providing a description and annotations, for example by automatically including text boxes, arrows, and lines around the relevant places that may only require re-adjustment by the operator. This is particularly useful, for example, if an assistant is doing only an intermediate examination. Then the pathologist, who is instructed with the final examination, can easily retrieve the information of the heat map and focus on the already identified interesting areas of the image. Thereby, time can be saved during analysis. The heat map results may be very useful in large university hospitals where an intermediate gross examination of histological specimens is typically performed by pathology residents or pathologists' assistants, but a final check is completed by experienced staff.

According to a preferred embodiment, the eye-tracking device may comprise infrared illuminators, cameras, and an image processing unit. Preferably, the imaging processing unit is configured to run an image processing algorithm.

Thereby, the eye-tracking device may be provided in the system in a particular efficient and cost effective manner.

According to a preferred embodiment, the eye-tracking device may be a stationary or portable or semi-portable device.

Thereby, the usability and versatility of the system is improved as the system can be used at different locations and in different environments.

A second aspect of the present invention relates to a method for the control of a system for medical gross examination. The method comprises the steps of placing at least one biospecimen to be examined in an examination area, visually inspecting the examination area by (at least one) a camera device and providing images captured during visual inspection for transmission, displaying the captured images on (at least one) a display device, detecting eye information based on the point of gaze and/or eye movement of a user's eye by (at least one) an eye-tracking device, and controlling the system by (at least one) a controller based on the detected eye information.

Thereby, it can be achieved that the operator's manual work is reduced to work relating only to contacting the biospecimen directly. Moreover, it can be avoided to handle and operate manually other equipment as the control thereof is completed by using a control based on eye information. Thus, the cleaning process and risk of contamination is reduced significantly and the quality and reliability of the analysis results can be improved.

Preferably, the method may also comprise the step of providing the system for the medical gross examination according to the first aspect of the present invention.

According to a preferred embodiment, in the method the eye information is associated to a defined reference object, e.g. the display device, and preferably to dedicated areas of the reference object, of which preferably each relates to a defined control function of the system. The control function may preferably be activated and parametrized based on the eye information. Alternatively or additionally, the method may also comprise the step of associating the eye information with a defined reference object.

Thereby, a correlation between the eye information and a (known) reference object can be established. Consequently, the evaluation of the eye information can be improved and the eye information can be used to improve the analysis and documentation process during the gross examination.

According to a preferred embodiment, the method may further comprise the step of creating a heat map based on the eye information, preferably based on detected eye positions as the eye information and being associated with relative or absolute durations recorded for each of the detected eye positions. Therein, the eye positions may preferably relate to the associated defined reference object, and more preferred to at least part of the dedicated areas. Preferably, the heat map may be created based on the duration recorded for each of the detected eye positions with respect to the dedicated areas of the displayed captured image so that the dedicated areas are provided with (different) colours that depend on the recorded duration of the detected eye position correlating with the respective detected area.

Thereby, it can be achieved to combine the analysis and examination process of the biospecimen directly with the documentation process. Thus, a significant time reduction for the gross examination can be achieved as relevant parts of the captured images can be highlighted.

Further features, advantages and objects of the present invention will become apparent for the skilled person when reading the following detailed description of embodiments of the present invention and when taking in conjunction with the figures of the enclosed drawings.
- **Figure 1**: shows a first embodiment of the system according to the invention.
- **Figure 2**: shows a second embodiment of the system according to the invention.
- **Figure 3**: shows a perspective view of a user with a first embodiment of an eye-tracking device and a display device of the invention.
- **Figure 4**: shows a front view of a second embodiment of an eye-tracking device and a display device of the invention.
- **Figure 5**: shows a front view of a third embodiment of an eye-tracking device and a display device of the invention.
- **Figure 6**: shows a perspective view of a fourth embodiment of a display device of the invention.

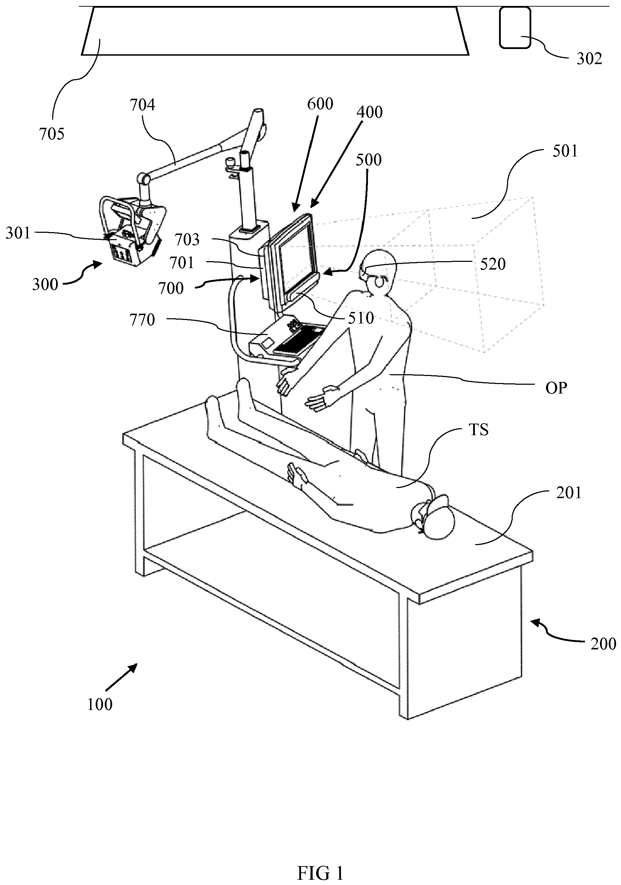

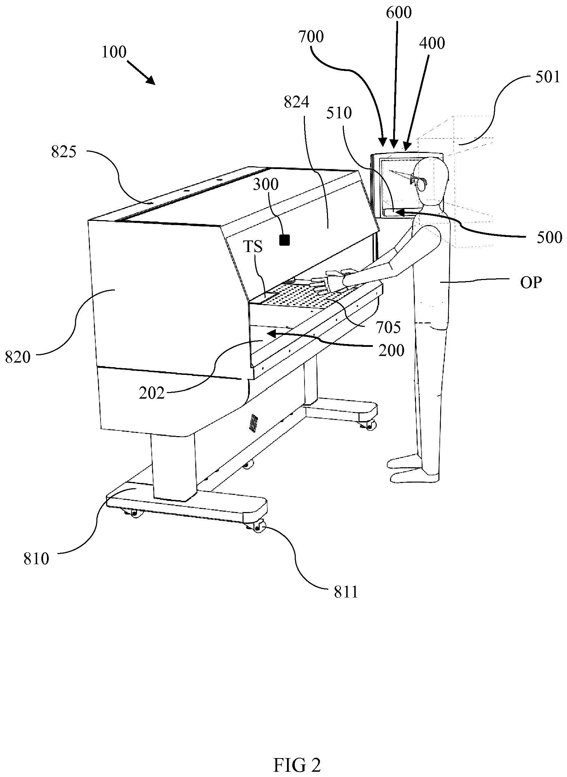

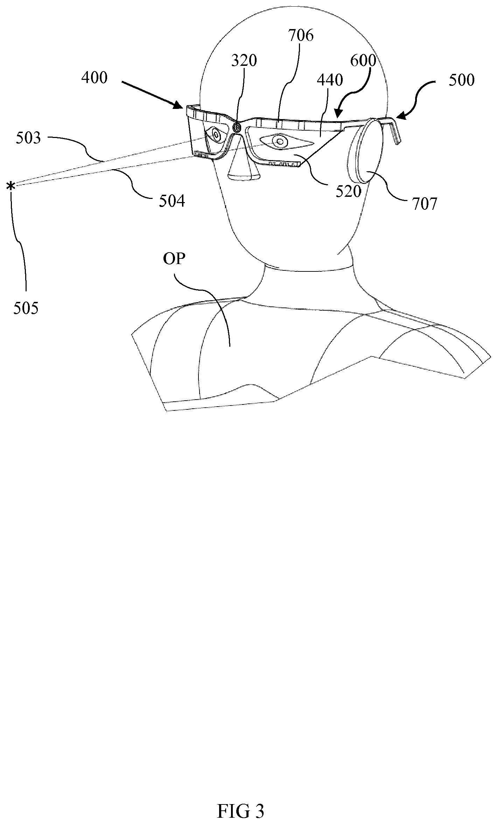

Figures 1 and 2 show different embodiments of a system 100 for medical gross examination according to the present invention. Figures 3 to 6 show different elements of the system 100 of the present invention in different views.

The system 100 comprises an examination area 200 for receiving biospecimens TS to be examined. Figures 1 and 2 show different embodiments of the examination area 200. For example, the examination area 200 in Figure 1 is a surgical or autopsy table 201 that may be suitable and/or configured to receive a(n) (entire) human body as the biospecimen TS. In Figure 2, the examination area 200 is formed by a laboratory working surface 202 that may be suitable and/or configured to receive organs or small tissue samples as the biospecimen TS. However, this enumeration is not complete and other examples of the examination area 200 exist. The examination area 200 may be configured to provide a surface that is sterile and/or easy to clean. For example, the examination area 200 may be provided with plastic matts that can be exchanged after an examination. The examination area 200 may be fixed to the ground of a room or may be movable. The examination area 200 may be provided in correlation with characteristics of the biospecimen TS. For example, the examination area 200 may have a colour that provides a (strong) contrast to the colour of the biospecimen TS.

The system 100 further comprises at least one camera device 300 for visual inspection of the examination area 200 and for providing images captured during visual inspection for transmission. The camera device 300 may have any size and form. Preferably, the camera device 300 may be a (digital) camera with a high resolution and high magnification capabilities for providing images of high quality. The camera device 300 may comprise a camera interface port for transmitting the captured images and/or for receiving a control signal for controlling the camera device 300 (remotely). The camera device 300 may be provided in the system 100 movable with respect to the examination area 200. The system 100 may comprise more than one of the described camera devices 300. This is exemplarily illustrated in Figure 1. For example, a first camera device 301 may be portable and/or movable with respect to the examination area 200. A second camera device 302 may be fixed to the ceiling or a wall of a room for providing a view of the examination area 200 from above. The camera device 300 may be fully enclosed in a casing. Moreover, it is also conceivable that the camera device 300 is integrated in objects worn by the operator OP, such as spectacle frames. This is exemplarily illustrated in Figure 3, which shows a miniature camera 320 being integrated in spectacle frames. Alternatively or additionally, the camera device 300 may also be worn around the head of the operator OP. The camera device 300 may be integrated in the examination area 200. Moreover, the camera device 300 may be suitable also for recording video streams and audio signals. However, this enumeration is not complete and further examples for the camera device 300 may be considered.

The system 100 further comprises at least one display device 400 for displaying the captured images. For example, the display device 400 may be a computer monitor or touch screen monitor, such as exemplarily illustrated in Figures 1, 2, 4 to 6, and/or a (LCD) projector. However, this enumeration is not complete and other examples are possible. The display device 400 may have any size and form. For example, the display device 400 may also be provided in objects worn by the operator OP, such as lenses 440 of spectacles. Figure 3 exemplarily illustrates the use of the lenses 440 as the display device 400. The display device 400 may be movable with respect to the camera device 300 and/or to the examination area 200. Preferably, the display device 400 can be positioned in the system 100 such that it enables the operator OP to view the display device 400 when being in a sitting or standing position. The display device 400 may have a display interface port for receiving information to be displayed. For example, the display device 400 may receive the captured images transmitted by the camera device 300 via its camera interface port through the display device's 400 display interface port. For this, the display device 400 may communicate with the camera device 300 over a wired or wireless (data) connection, for example using a wireless internet connection. The display device 400 may be integral with the camera device 300.

The system 100 may further comprise at least one system component 700 for providing additional functionality in the system 100.

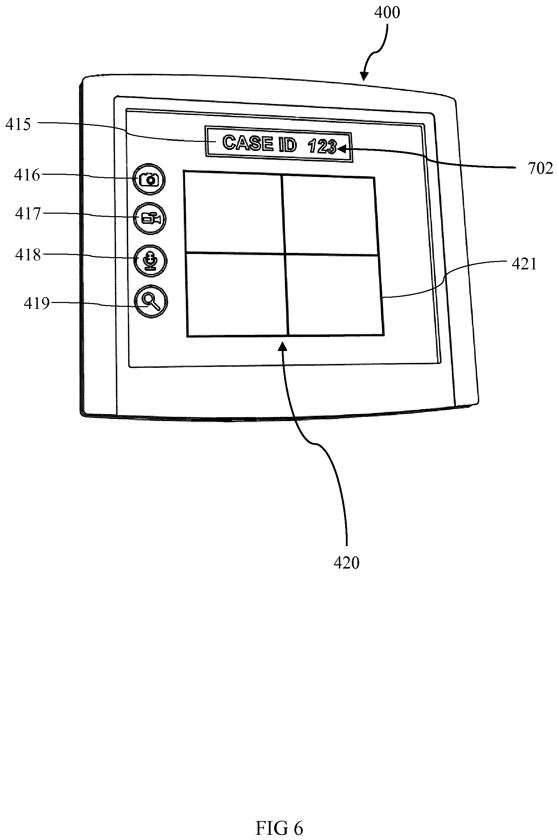

The component 700 may be a data system 701 for storing data collected during gross examination (gross examination data). For example, the data system 701 may be a database. Thereby, an image database, considered an indispensable resource for quality assurance (QA) and accreditation, can be created. The stored images form a powerful database for QA, teaching and research. The data system 701 is exemplarily illustrated in Figure 1 as being integrated in the display device 400. However, the data system 701 may be also provided as a (structurally) separate component of the system 100. Preferably, the gross examination data may be linked to a unique data code 702. The unique data code 702 may be a case ID, such as exemplarily illustrated in Figure 6. Thereby, storing and finding data in the data system 701 may be simplified.

Moreover, the component 700 may be an internet device 703 for accessing external data. For example, the internet device 703 may be configured to access an external main data system, such as a Laboratory Information System (LIS). Thereby, exchanging data collected in the system 100 and external data systems can be facilitated. Moreover, the internet device 703 may allow to connect to remote transmission facilities for teleconferencing and telepathology consultations. It is conceivable that the internet device 703 is integrated in the display device 400 such as exemplarily illustrated in Figure 1. However, the internet device 703 may be also provided as a (structurally) separate component of the system 100.

The component 700 may be a communication device 707, like a telephone device or a VoIP device, for external communication, and/or a microphone device 706 for recording an audio signal. This is exemplarily illustrated in Figure 3. The communication device 707 is attached to spectacle frames that also comprise the microphone device 706. However, it is also conceivable that the communication device 707 or the microphone device 706 are integrated in the display device 400 or that they are provided as (structurally) separate components.

Moreover, the component 700 may be a robot arm device 704 for handling biospecimens TS into and out of the examination area 200. Moreover, the robot arm device 704 may be provided also for handling biospecimens TS at the examination area 200. The robot arm device 704 may be pivotable around a main axis and may be provided such that it is relatively moveable with respect to the examination area 200. For example, Figure 1 exemplarily illustrates the system 100 comprising the robot arm device 704, to which the camera device 300 (and/or the display device 400) attaches for assisting the operator OP in moving the camera device 300 relatively to the examination area 200 (and/or the display device 400). The robot arm device 704 may comprise servomotors and actors in order to facilitate controlled automated movement thereof. The robot arm device 704 may be provided on wheels or fixed to the floor of the laboratory.

The component 700 may be a ventilation device 705 for ventilation of the examination area 200 in order to extract dangerous fumes. For example, the cutting activities may involve for the operator OP a continuous risk of inhalation of noxious vapours, for example of formaldehyde. The ventilation device 705 may be provided integrally with the examination area 200. This is exemplarily illustrated in Figure 2, which shows that the ventilation device 705 is configured to immediately extract any fumes by drawing air downwards into holes provided in the examination area 202. Alternatively or additionally, the ventilation device 705 may be provided on a ceiling or wall of the room such as exemplarily illustrated in Figure 1.

The system 100 may also be provided with a laboratory hood 820, such as illustrated in Figure 2, for safely containing and/or removing hazardous biological and chemical fumes before they can escape into the lab or environment. The laboratory hood 820 may be provided with openings 825 to release filtered air into the environment.

The laboratory hood 820 may be a spanning benchtop model or a walk-in unit. The hood 820 may be a ducted hood for removing contaminants by using constant and/or variable air volume. Preferably, the laboratory hood 820 may be a low-flow unit that incorporates fume containment features to save energy while maintaining safety.

The system 100 may be provided as being moveable or static. Figures 1 and 2 exemplarily display two movable embodiments. For example, in Figure 2 the hood 820 is connected to a movable platform 810, which comprises wheels 811 for enabling rolling movement thereof. Similarly, in Figure 1 the robotic arm device 704 may be provided on wheels. However, it is also conceivable that each of the systems 100 illustrated in these figures may be provided statically.

The hood 820 may comprise a transparent cover. The transparent cover 824 may comprise the display device 400 and/or the camera device 300. The transparent cover 824 may be provided for directing the (flow of) fumes away from the operator OP.

The system 100 further comprises an eye-tracking device 500 for detecting eye information based on the point of gaze 505 and/or eye movement of a user's OP eye.

The point of gaze 505 may be understood as a present location, in which the eye(s) of the operator OP is focused (momentarily). This is exemplarily illustrated in Figure 3, where the operator OP gazes towards an object in front and corresponding lines of sight 503, 504 are (bundled) focused in the point of gaze 505.

Preferably, the eye-tracking device 500 may be configured to carry out measurements, in which the position of the eyes, their movements and/or the point of gaze 505 are/is recorded, preferably in relation to the environment. Preferably, the eye-tracking device 500 may be configured to optically track corneal reflections (also known as Pupil Centre Corneal Reflection (PCCR)) to facilitate such measurements.

Preferably, the eye-tracking device 500 may comprise (an) infrared illuminator(s), (an) (infrared) camera(s), and an image processing unit. The imaging processing unit may be preferably configured to run an image processing algorithm.

The infrared illuminators may be provided to direct (near-)infrared light towards the centre of the eyes (pupil). This is exemplarily illustrated in Figures 1 and 2 by space 501, which is illuminated by infrared light by the illuminators.

The infrared light may cause visible reflections in the cornea (outer-most optical element of the eye) that are tracked by the infrared camera.

In particular, the illuminators may create a pattern of (near-)infrared light on the eyes. The cameras may then take images of the user's OP eye(s) and the reflected patterns. The image processing unit may be configured to calculate the eyes' position and point of gaze 505 and/or find specific details in the user's OP eyes and reflections patterns.

Infrared light may be preferred as the accuracy of the gaze direction measurement may be improved due to a clear demarcation of the pupil and the detection of corneal reflection. Other light sources (and non-infrared cameras) may be less suited for providing sufficient contrast. Also, the visible spectrum is likely to generate uncontrolled specular reflection, while infrared light allows for a precise differentiation between the pupil and the iris. Thus, the accuracy of the eye-tracker 500 may be improved by using infrared light as light source. Additionally, infrared light does not cause any distraction while the eyes are being tracked as it is not visible to humans. However, the use of other light sources may also be considered.

The eye-tracking device 500 may be a stationary or portable or semi-portable device. Figures 1 and 2 show examples for stationary eye-tracking devices 510.

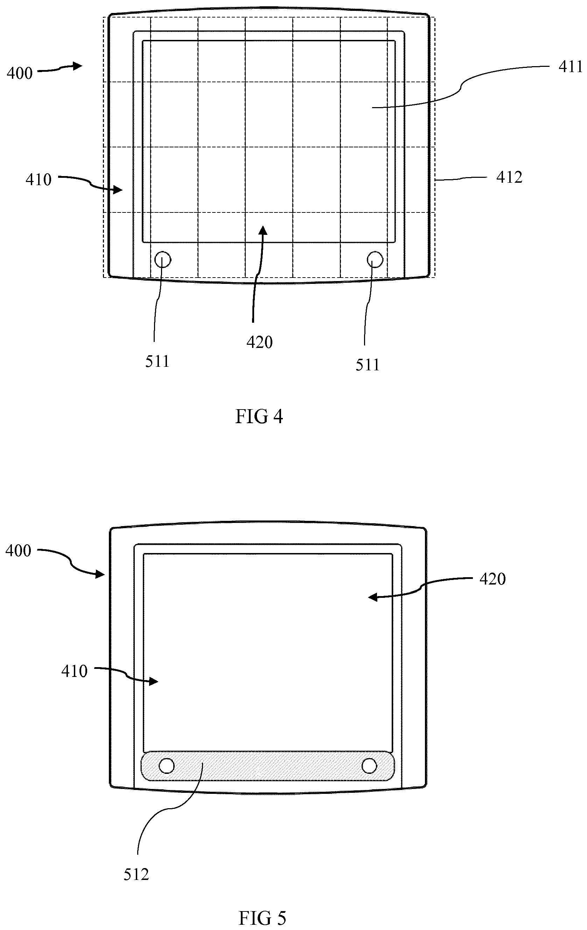

Preferably, in a stationary setup, the eye-tracking device 500 may be positioned near the object to be tracked. Preferably, the object to be tracked may be the display device 400 and the operator OP may be in front of the display device 400. Thereby, the freedom of movement of the operator OP is still sufficiently large for being relatively unrestricted while achieving precise observations of two-dimensional structures. The eye-tracking device 500 may be integrated (built in) into the display device 400. This is exemplarily illustrated for the display device 400 in Figure 4, in which the eye-tracking device 511 is built into the display device 400. Alternatively or additionally, (the infrared illuminator and camera of) the eye-tracking device 500 may be integrated in a PC or laptop.

Alternatively, the eye-tracking device 500 may be detachably connected to the display device 400. This is exemplarily illustrated in Figure 5, in which the tracking device 512 is connected via mounting portions to the display device 400. It is also conceivable that infrared illuminators and camera may be provided separately to a PC or laptop.

Figure 1 and 3 show examples for a portable eye-tracking device 520. In these two figures, the eye-tracking device 500 is integrated into a spectacle frame. Preferably, the spectacles used in Figures 1 to 3 can also be used as PPE-equipment. A portable setup is particularly helpful if the object to be observed has a three-dimensional structure, or if the operator needs to be able to move freely. Therein, the infrared illuminator and camera may be preferably integrated in wearable objects, such as a spectacle as illustrated exemplarily in Figure 3.

A further advantage of this setup may be that eye-tacking is conducted directly in relation to scenes (images) seen by the operator OP. Thus, the recording made by the camera device 300 and by the eye-tracking device 500 are (directly) interlinked and/or connected.

Naturally, it is also conceivable that the system 100 comprises more than one eye-tracking device 500, such as exemplarily illustrated in Figure 1 by the stationary eye-tracking device 510 and the portable eye-tracking device 520.

The system 100 further comprises a controller 600 for controlling the system 100 based on the eye information detected by the eye-tracking device 500.

The controller 600 may be, for example, a computer or a microcontroller. Preferably, the controller 600 may also comprise the image processing unit of the eye-tracking device 500. The controller 600 may be provided together with the display device 400 as one single unit such as illustrated exemplarily in Figures 1 to 3. However, it is also conceivable that the controller 600 may be provided separately.

The controller 600 may be configured to control the system 100 based on the eye information detected by the eye-tracking device 500.

The controller 600 may be configured to activate defined control functions based on the eye information, wherein preferably parameters of the defined control functions are based on or set via the eye information. The defined control functions are illustrated exemplarily as icons 415 to 419 on the display device 400 in Figure 6.

For example, (one of) the defined control functions may be a camera device function 416 that may enable a zoom-in/zoom-out function, an image capturing function, and/or a video recording function. Corresponding parameters of the camera device function 416 may be a zoom factor or a start/stop command for video recording.

Moreover, (one of) the defined control functions may be a display device function 417 that may allow changing a resolution of the display device 400 or the content to be displayed on the display device 400, and/or editing of the displayed captured images. Corresponding parameters of the display device function 417 may be a text to be added or a name of a file to be opened.

Further, (one of) the defined control functions may be a data system function 419 that enables accessing the data system 701 for retrieving and storing any data, e.g. data that is displayed on the display device 400. Alternatively or additionally, the data system function 419 may enable to search and open cases displayed on the display device 400. The unique data code 702 of a case may be used as a parameter of this function. Indicator function 415 may display the unique data code 702.

In addition, the defined control functions may comprise an internet access function for accessing external data, a data analysis function for analysing and evaluating data, a communication function for establishing a communication link, a microphone device function 418 for starting and stopping an audio recording, and a ventilation function for controlling the configuration of the ventilation device 705, such as setting an air volume to be extracted.

Further, (one of) the defined control functions may be a robot arm function for manipulating/handling biospecimen TS and/or the camera device 300. Therein, parameters of this function may be a direction of movement or new position for the robot arm device 704.

Further, the controller 600 may be configured such that activating at least one of the control functions activates at least one other control function to be activated by the eye information, wherein the at least one activated control function is preferably displayed on the display device 400 for being operated for preferably a predetermined time only. For example, starting a video recording by activating the camera device function 416 may also activate the microphone device function 418 to include documentation in the video recording. In addition, the controller 600 may be configured to control or to allow control of at least one of the control functions or of the other control functions for a predetermined time only.

The controller 600 may be configured such that the eye information is associated with a defined reference object, e.g. the display device 400. Preferably, the eye information may be associated with dedicated areas 410 of the reference object. In Figure 4, the dedicated areas 410 are exemplarily illustrated as individual fields.

Figure 6 illustrates a further example, in which each of the dedicated areas 410 may relate to at least one of the defined control functions of the system 100. For example, the dedicated areas 410 may be functional areas 415-419, such as icons, which relate to or display functions of the system 100 to be operated. For example, icon 416 relates to a control function of the camera device 300, icon 417 relates to a control function of the display device 400, icon 418 relates to a control function of the microphone device and icon 419 relates to a control function of the data system 701. Naturally, it is also conceivable that functional areas are provided with respect to control functions of the internet device 703, the communication device, the robot arm device 704 and/or the ventilation device 705.

For example, activation of the defined control functions may be effected by resting the eyes for a predetermined time period on one of the dedicated areas 410, in particular the functional areas 415-419 illustrated exemplarily in Figure 6.

Alternatively or additionally, the dedicated areas 410 may comprise defined spatial areas of the reference object, like a section 411 of a raster 412. This is exemplarily illustrated in Figure 4, where the dedicated areas 410 may comprise defined areas on the display device 400 that are divided in the raster 412 with raster sections 411. The dedicated areas 410 may also be an image display area 420 for displaying the captured image on the display device 400. This is exemplarily illustrated in Figures 4 to 6. The image display area 420 may be a defined area of the display device 400 that is provided for displaying the captured images. The image display area 420 may be preferably being divided into sub-areas 421. The dedicated areas 410 may also include an input area for inputting and/or storing information in the system 100.

The controller 600 may be configured to create a heat map based on the eye information. The heat map may be preferably based on detected eye positions (used by the controller 600 as the eye information), which are associated with relative or absolute durations recorded for each of the detected eye positions. The eye positions may preferably relate to the associated defined reference object, and more preferred to at least part of the dedicated areas 410. The controller 600 may be configured to create the heat map based on the recorded duration of each of the detected eye positions with respect to the dedicated areas 410 of the displayed captured image. The controller 600 may be configured to create the heat map with different colours for the respective dedicated areas 410, wherein each different colour represents a different duration or duration range recorded for the detected eye position correlating with the dedicated areas 410.

The system 100 may also be (specifically) configured for conducting a medical gross examination. Moreover, the system 100 may also be combined with traditional input means, including a keyboard 770, mouse, trackpad, foot pedal and/or voice control. This is exemplarily illustrated in Figure 1.

A further aspect of the present invention is directed to a method for the control of a system for medical gross examination.

Preferably, the method further comprises as a first step the step of providing the system 100 as described in detail before.

The method comprises the steps of placing at least one biospecimen TS to be examined in the examination area 200, visually inspecting the examination area 200 by the camera device 300, providing images captured during visual inspection for transmission, displaying the captured images on the display device 400, detecting eye information based on the point of gaze 505 and/or eye movement of a user's OP eye by the eye-tracking device 500, and controlling the system 100 by a controller 600 based on the detected eye information.

The order of the steps may be interchangeable. However, benefits may arise from following the order of steps as described hereinbefore.

Preferably, the eye information used in the method for control of the respective system is associated to a defined reference object, e.g. the display device 400, and preferably to dedicated areas 410 of the reference object, of which preferably each relates to a defined control function of the system 100 that is preferably activated and parametrized based on the eye information.

The method may further comprise the step of creating a heat map based on the eye information or based on detected eye positions as the eye information. The eye information may be is associated with relative or absolute durations recorded for each of the detected eye positions. Therein, the eye positions may preferably relate to the associated defined reference object, and more preferred to at least part of the dedicated areas 410. Thus, the controller 600 may be provided with a counter/timer.

In particular, the heat map may be created based on the duration recorded for each of the detected eye positions with respect to the dedicated areas 410 of the displayed captured image. Thereby, each of the dedicated areas 410 can be provided in different colours that depend on the recorded duration of the respective detected eye position.

The method may further comprise a step of confirming the identity of the patient and the anatomical site from which the biospecimen TS was obtained. Such information may be necessary in order to guide the operator OP to the appropriate diagnostic examination and to facilitate a correct interpretation of the biospecimen. The operator OP may then enter biospecimen/patient data by scanning a barcode or entering the unique data code 702 connected to the biospecimen TS or by activating the data system function 419 for retrieval thereof.

Once the biospecimen TS is situated on the examination area 200 it may be possible to generate the gross description. It may be possible/necessary to enhance/create this diagnostic report by using the defined control functions.

The present invention is not limited by the embodiments as described hereinabove, as long as being covered by the appended claims. All the features of the embodiments described herein above can be combined in any possible way and can be interchangeably provided.

## Claims

1. System (100) for medical gross examination, comprising:
• an examination area (200) for receiving biospecimens (TS) to be examined,
• a camera device (300) for visual inspection of the examination area (200) and for providing images captured during visual inspection for transmission,
• a display device (400) for displaying the captured images,
• an eye-tracking device (500) for detecting eye information based on the point of gaze (505) and/or eye movement of a user's (OP) eye, and
• a controller (600) for controlling the system (100) based on the eye information detected by the eye-tracking device (500).

2. System (100) according to claim 1, wherein the system (100) further comprises at least one system component (700) that comprises at least one or more or all of the group consisting of:
• a data system (701) for storing gross examination data, preferably linked to a unique data code (702),
• an internet device (703) for accessing external data, preferably an external main data system,
• a communication device (707), like a telephone device or a VoIP device, for external communication,
• a microphone device (706) for audio recording,
• a ventilation device (705) for ventilation of the examination area (200).

3. System according to claim 1 or 2, wherein the controller (600) is configured to activate defined control functions based on the eye information, wherein preferably parameters of the defined control functions are based on or set via the eye information.

4. System (100) according to claim 3, wherein the defined control functions comprise at least one or more or all of the group consisting of:
• a camera device function (416), such as a zoom-in/zoom-out function, an image capturing function, and/or a video recording function;
• a display device function (417), such as changing a resolution of the display device (400) or content to be displayed, and/or editing of the captured images;
• a data system function (419), such as accessing the data system (701) for retrieving and storing data, e.g. data as displayed on the display device (400), and/or for searching and opening cases to be displayed on the display device (400);
• an internet access function for accessing external data;
• a communication function for establishing a communication link;
• a microphone device function (418), such as starting and stopping an audio recording;
• a ventilation function for controlling the configuration, e.g. the power, of the ventilation device (705);
• a data analysis function for analyzing and evaluating data.

5. System (100) according to claims 3 or 4, wherein the controller (600) is configured such that activating at least one of the control functions activates at least one other control function to be activated by the eye information, wherein the at least one activated control function is preferably displayed on the display device (400) (for being operated) or more preferred at the dedicated areas (410), preferably for a predetermined time only.

6. System (100) according to any one of the preceding claims 3 to 5, wherein the controller (600) is configured to control or to allow control of at least one of the control functions or of the other activated control functions for a predetermined time only.

7. System (100) according to any one of the preceding claims, wherein the controller (600) is configured such that the eye information is associated with a defined reference object, e.g. the display device (400), and preferably with dedicated areas (410) of the reference object, wherein preferably each of the dedicated areas (410) relates to at least one of the defined control functions of the system (100).

8. System (100) according to claim 7, wherein the dedicated areas (410) comprise at least one or more or all of the group consisting of:
• defined spatial areas of the reference object, like a section (411) of a raster (412),
• defined areas on the display device (400), preferably divided in a raster (412) having raster sections (411),
• an image display area (420), preferably being divided into sub-areas, for displaying the captured image on the display device (400),
• functional areas (415-419) related to or displaying functions of the system (100) to be operated, like control functions of
∘ the camera device (300);
∘ the display device (400);
∘ the data system (701);
∘ the internet device (703);
∘ the communication device (707);
∘ the microphone device (706);
∘ the ventilation device (705);
• an input area for inputting and/or storing information in the system (100), preferably the data system.

9. System (100) according to any one of the preceding claims, wherein the controller (600) is configured to create a heat map based on the eye information, preferably based on detected eye positions as the eye information and being associated with relative or absolute durations recorded for each of the detected eye positions, wherein the eye positions preferably relate to the associated defined reference object, and more preferred to at least part of the dedicated areas (410).

10. System (100) according to claim 9, wherein the controller (600) is configured to create the heat map based on the recorded duration of each of the detected eye positions with respect to the dedicated areas (410) of the displayed captured image, and/or
wherein the controller (600) is configured to create the heat map with (different) colours for the respective dedicated areas (410), wherein different colours represent different durations or duration ranges recorded for the detected eye positions correlating with the dedicated areas (410).

11. System (100) according to any one of the preceding claims, wherein the eye-tracking device (500) comprises infrared illuminators, cameras, and an image processing unit, wherein the imaging processing unit is preferably configured to run an image processing algorithm.

12. System (100) according to any one of the preceding claims, wherein the eye-tracking device (500) is a stationary or portable or semi-portable device.

13. Method for the control of a system (100) for medical gross examination, comprising the steps of:
• placing at least one biospecimen (TS) to be examined in an examination area (200),
• visually inspecting the examination area (200) by a camera device (300) and providing images captured during visual inspection for transmission,
• displaying the captured images on a display device (400),
• detecting eye information based on the point of gaze (505) and/or eye movement of a user's (OP) eye by an eye-tracking device (500), and
• controlling the system (100) by a controller (600) based on the detected eye information.

14. Method according to claim 13, wherein the eye information is associated to a defined reference object, e.g. the display device (400), and preferably to dedicated areas (410) of the reference object, of which preferably each relates to a defined control function of the system (100) that is preferably activated and parametrized based on the eye information.

15. Method according to claim 13 or 14, further comprising the step of creating a heat map based on the eye information, preferably based on detected eye positions as the eye information and being associated with relative or absolute durations recorded for each of the detected eye positions, wherein the eye positions preferably relate to the associated defined reference object, and more preferred to at least part of the dedicated areas (410),
wherein the heat map is preferably created based on the duration recorded for each of the detected eye positions with respect to the dedicated areas (410) of the displayed captured image so that the dedicated areas (410) are provided with (different) colours that depend on the recorded duration of the detected eye position correlating with the respective detected area.
